(19) Europäisches Patentamt
European Patent Office
Office européen des brevets

(11) EP 1 449 831 A1

(12) # EUROPEAN PATENT APPLICATION

(43) Date of publication:
**25.08.2004 Bulletin 2004/35**

(51) Int Cl.[7]: **C07D 203/24**

(21) Application number: **04250770.7**

(22) Date of filing: **12.02.2004**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **12.02.2003 JP 2003033351**
**31.07.2003 JP 2003204553**

(71) Applicant: **Kyushu University**
**Fukuoka City, Fukuoka Pref. (JP)**

(72) Inventors:
- **Katsuki Tsutomu, c/o Kyushu University**
  **Fukuoka City, Fukuoka Pref. (JP)**
- **Omura, Kazufumi**
  **Fukuoka City, Fukuoka Pref. (JP)**
- **Murakami, Masakazu**
  **Ichihara City, Chiba Pref. (JP)**
- **Uchida, Tatsuya**
  **Fukuoka City, Fukuoka Pref. (JP)**
- **Irie, Ryo, c/o Kyushu University**
  **Fukuoka City, Fukuoka Pref. (JP)**

(74) Representative: **Whalley, Kevin**
**MARKS & CLERK,**
**57-60 Lincoln's Inn Fields**
**London WC2A 3LS (GB)**

(54) **Method of producing optically active aziridine compounds and amine compounds**

(57) An optically active aziridine compound or amine compound is produced by using a specified Ru(salen)(CO) complex as a catalyst, and subjecting a specified olefin to an asymmetric aziridination or amination with a specified arylsulfonyl azide compound.

EP 1 449 831 A1

**Description**

BACKGROUND OF THE INVENTION

1. Field of the Invention

This invention relates to a method of producing optically active aziridine compounds and amine compounds. Such optically active aziridine compounds and amine compounds can be used for the synthesis of medicines and agrochemicals.

2. Description of the Related Art

Nitrene transfer is a fundamental C-N bond formation reaction. It is well-known that many metal complexes catalyze the nitrene transfer reaction via a metal nitrenoide intermediate, and much effort has been directed to asymmetric nitrene transfer reactions, especially aziridination and amination. Chiral metal complexes such as metalloporphyrins, metallosalens, Cu-bis(oxazoline), Cu-bis(Schiff base), Cu-diamine complexes and the like have been examined as a catalyst for the asymmetric nitrene transfer reactions, and it has been reported that they have a high enantioselectivity.

However, a reagent being not efficient in terms of atom economy such as arylsulfonyliminophenyliodinane ($PhI=NSO_2Ar$) or the like is used as a nitrene precursor in the above reports, so that there is a problem in the economical efficiency of the production process.

On the other hand, N-arylsulfonyl azides and N-carbamoyl azides are known as a very economical nitrene precursor. Jacobsen et al. reported that N-arylsulfonyl azide serves as a nitrene precursor for an asymmetric aziridination in the presence of copper ion under photo-irradiation. Back et al. reported a sulfimidation using N-t-butoxycarbonyl azide. Further, the inventors reported that an alkyl aryl sulfide can be subjected to an asymmetric sulfimidation by using N-arylsulfonyl azide as a nitrene precursor in the presence of a Ru(salen)(CO) complex without photo-irradiation (M. Murakami, T. Uchida and T. Katsuki, Tetrahedron Letters, vol. 42, page 7071 (2001)).

However, there are not yet reported examples of conducting asymmetric aziridination and amination with high enantioselectivity by using economical nitrene precursors.

SUMMARY OF THE INVENTION

It is, therefore, an object of the invention to provide a method of producing optically active aziridine compounds and amine compounds with high enantioselectivity by using an available and economical nitrene precursor.

The inventors have made various studies in order to achieve the above object and discovered that an aziridine compound or an amine compound having a high optical purity can be produced by using a specified Ru(salen)(CO) complex as a catalyst and subjecting specified olefins to an asymmetric aziridination or amination with arylsulfonyl azide compounds, and as a result, the invention has been accomplished.

According to the first aspect of the invention, there is the provision of a method of producing an optically active aziridine compound represented by the following formula (I):

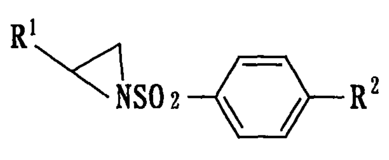

· · · · · (I)

(wherein $R^1$ is an alkenyl group having a carbon number of 2 to 20, an alkynyl group having a carbon number of 2 to 20 or an aryl group having a carbon number of 6 to 20 provided that a hydrogen atom in these alkenyl group, alkynyl group and aryl group may be substituted with a halogen atom or a nitro group, and $R^2$ is a hydrogen atom, a halogen atom, a substituted or non-substituted alkyl group having a carbon number of 1 to 4, or a substituted or non-substituted alkoxy group having a carbon number of 1 to 4), which comprises using as a catalyst an optically active Ru(salen)(CO) complex represented by the following formula (II) or (III):

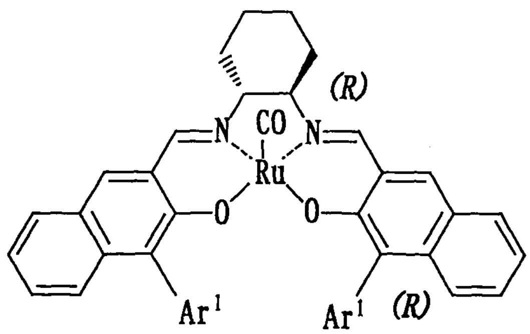

· · · · · (II)

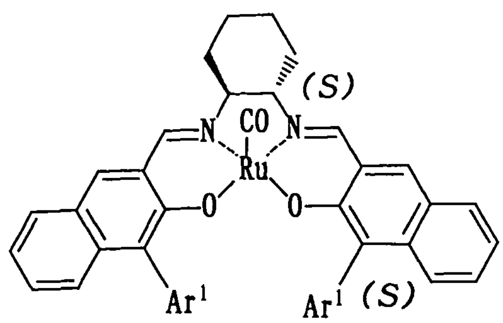

· · · · · (III)

(wherein $Ar^1$ is independently an aryl group having a carbon number of 10 to 16), and subjecting an olefin represented by the following formula (IV):

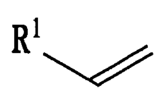

· · · · · (IV)

(wherein $R^1$ is the same meaning as mentioned above) to an asymmetric aziridination with an arylsulfonyl azide compound represented by the following formula (V):

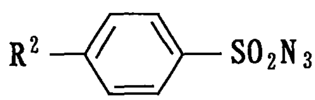

· · · · · (V)

(wherein $R^2$ is the same meaning as mentioned above).

In a preferable embodiment of the first aspect of the invention, the Ru(salen)(CO) complex is represented by the following formula (VI) or (VII).

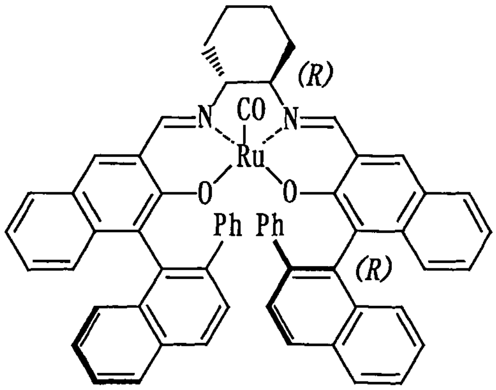

····· (VI)

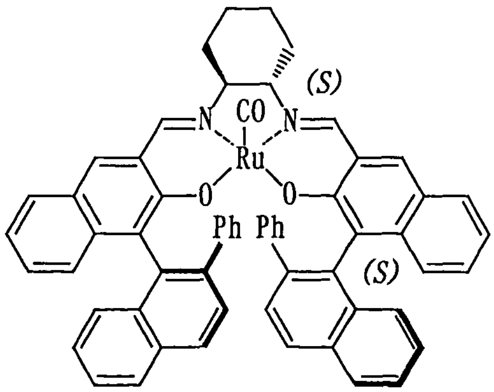

····· (VII)

In another preferable embodiment of the first aspect of the invention, $R^1$ in the olefin of the formula (IV) is phenyl group, p-bromophenyl group, p-nitrophenyl group, phenylethynyl group, 2-naphthyl group, 1-phenylvinyl group or p-(1-cyclohexenyl)-phenyl group.

In the other preferable embodiment of the first aspect of the invention, the arylsulfonyl azide compound of the formula (V) is p-toluenesulfonyl azide ($P-CH_3C_6H_4SO_2N_3$).

According to the second aspect of the invention, there is the provision of a method of producing an optically active amine compound represented by the following formula (VIII):

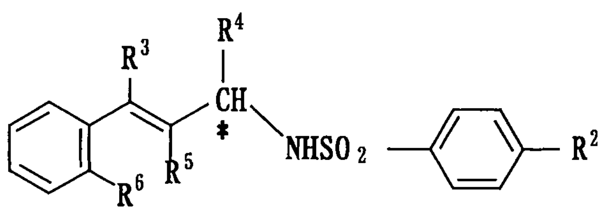

····· (VIII)

(wherein $R^2$ is a hydrogen atom, a halogen atom, a substituted or non-substituted alkyl group having a carbon number of 1 to 4, or a substituted or non-substituted alkoxy group having a carbon number of 1 to 4, and $R^3$, $R^4$, $R^5$ and $R^6$ are independently a hydrogen atom, or a linear or branched alkyl group having a carbon number of 1 to 20, provided that $R^3$ may combine with $R^4$ and $R^5$ may combine with $R^6$ to form a 5-member ring or a 6-member ring), which comprises using as a catalyst an optically active Ru(salen)(CO) complex represented by the following formula (II) or (III):

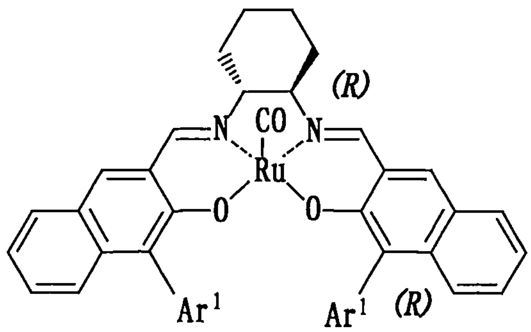

..... (II)

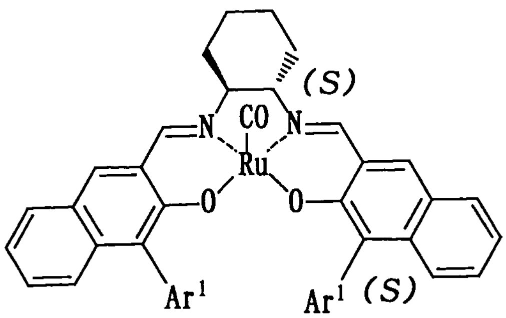

..... (III)

(wherein $Ar^1$ is independently an aryl group having a carbon number of 10 to 16), and subjecting an olefin expressed by the following formula (IX):

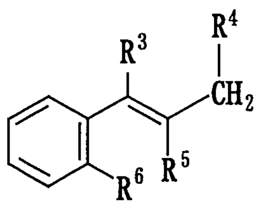

..... (IX)

(wherein each of $R^3$, $R^4$, $R^5$ and $R^6$ is the same meaning as mentioned above) to an asymmetric amination with an arylsulfonyl azide compound expressed by the following formula (V):

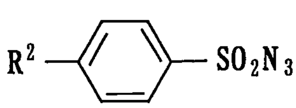

..... (V)

(wherein $R^2$ is the same meaning as mentioned above).

In a preferable embodiment of the second aspect of the invention, the Ru(salen)(CO) complex is represented by the following formula (VI) or (VII).

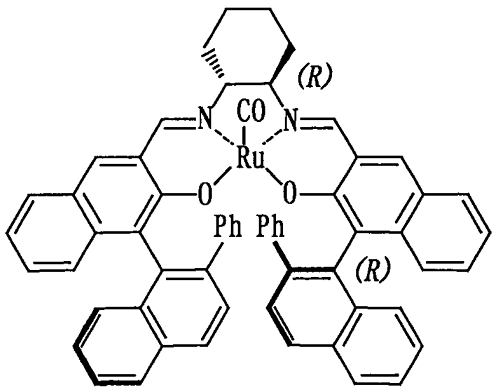

· · · · · (VI)

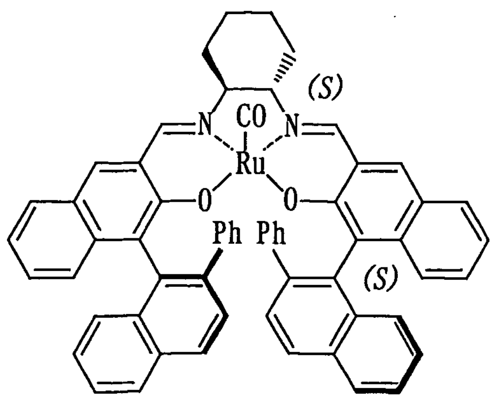

· · · · · (VII)

In another preferable embodiment of the second aspect of the invention, the olefin of the formula (IX) is represented by the following formula (X) or (XI).

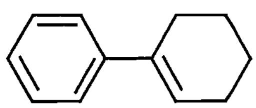

· · · · · (X)

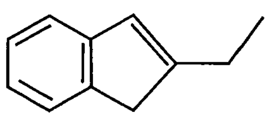

· · · · · (XI)

In the other preferable embodiment of the second aspect of the invention, the arylsulfonyl azide compound of the formula (V) is p-toluenesulfonyl azide (p-$CH_3C_6H_4SO_2N_3$).

DETAILED DESCRIPTION OF THE INVENTION

The invention will be explained in detail below. The optical active Ru(salen) complex used as a catalyst in the invention is represented by the formula (II) or (III). The complex of the formula (III) is an enantiomer of the complex of the formula (II). In the formulae (II) and (III), $Ar^1$ is independently an aryl group having a carbon number of 10 to 16. As the aryl group, mention may be made of 1-naphthyl group, 2-biphenyl group, 2-phenyl-1-naphthyl group, 2-methyl-1-naphthyl group, 2-(3,5-dimethylphenyl)-1-naphthyl group, 2-(4-methylphenyl)-1-naphthyl group, 2-[4-(t-butyldiphenylsilyl)phenyl]-1-naphthyl group, 2-methoxy-1-naphthyl group and so on. Among them, 2-phenyl-1-naphthyl group is particularly preferable. In the case that $Ar^1$ is 2-phenyl-1-naphthyl group, the Ru(salen)(CO) complex is represented by the formula (VI) or (VII). Moreover, the Ru(salen)(CO) complex has a CO ligand at its apical position. An amount of the Ru(salen)(CO) complex used as a catalyst is a range of 0.1 to 100 mol%, preferably 1 to 4 mol% per the molar amount of the olefin as a substrate.

The arylsulfonyl azide compound used as a nitrene precursor in the invention is represented by the formula (V). In this formula, $R^2$ is a hydrogen atom, a halogen atom, a substituted or non-substituted alkyl group having a carbon number of 1 to 4, or a substituted or non-substituted alkoxy group having a carbon number of 1 to 4. As the alkyl group having a carbon number of 1 to 4, mention may be made of methyl group, ethyl group, propyl group, isopropyl group, butyl group, isobutyl group, t-butyl group and so on. As the alkoxy group having a carbon number of 1 to 4, mention may be made of methoxy group, ethoxy group, propoxy group, butoxy group and so on. These alkyl groups and alkoxy groups may be substituted with a halogen and the like. Among the arylsulfonyl azide compounds, p-toluenesulfonyl azide (p-$CH_3C_6H_4SO_2N_3$), p-methoxybenzenesulfonyl azide (p-$CH_3OC_6H_4SO_2N_3$) and p-bromobenzene-sulfonyl azide (p-$BrC_6H_4SO_2N_3$) are preferable, and p-toluenesulfonyl azide (p-$CH_3C_6H_4SO_2N_3$) is particularly preferable. The arylsulfonyl azide compound can be easily synthesized from a commercially available arylsulfonyl chloride at one step (e.g., the synthesis process is described on page 505 of "Organic synthesis experiment handbook" published by Maruzen). An amount of the arylsulfonyl azide compound used is a range of 1 to 3 mol, preferably 1 to 1.5 mol per 1 mol of the olefin as a substrate. From a viewpoint that the yield is increased, it is preferable that the molar amount of the arylsulfonyl azide compound is made somewhat excess as compared with that of the olefin.

A complicatedly synthesized nitrene precursor such as arylsulfonyl-iminophenyliodinanes ($ArSO_2N{=}IPh$) or the like is used in the conventional nitrene transfer reaction. On the contrary, in the invention, an easily synthesized arylsulfonyl azide ($ArSO_2N_3$) is used as a nitrenre precursor, so that there are advantages that the production process can be simplified and the cost can be lowered.

In the invention, the olefin used as a starting material differs between the method of producing an optically active aziridine compound and the method of producing an optically active amine compound. That is, a conjugated terminal olefin represented by the formula (IV) is used in the method of producing an optically active aziridine compound, while a conjugated olefin having an allylic hydrogen represented by the formula (IX) is used in the method of producing an optically active amine compound.

In the olefin represented by the formula (IV), $R^1$ is an alkenyl group having a carbon number of 2 to 20, an alkynyl group having a carbon number of 2 to 20 or an aryl group having a carbon number of 6 to 20. As the alkenyl group having a carbon number of 2 to 20, mention may be made of 1-phenylvinyl group, 2-phenylvinyl group, isopropenyl group and so on. As the alkynyl group having a carbon number of 2 to 20, mention may be made of phenylethynyl group, trimethylsilylethynyl group, cyclohexylethynyl group and so on. As the aryl group having a carbon number of 6 to 20, mention may be made of phenyl group, 2-naphthyl group, p-(1-cyclohexenyl)-phenyl group, p-biphenyl group and so on. A hydrogen atom in these alkenyl groups, alkynyl groups and aryl groups may be substituted with a halogen atom or a nitro group.

On the other hand, in the olefin represented by the formula (IX), $R^3$, $R^4$, $R^5$ and $R^6$ are independently a hydrogen atom, or a linear or branched alkyl group having a carbon number of 1 to 20. As the linear or branched alkyl group having a carbon number of 1 to 20, mention may be made of methyl group, ethyl group, propyl group, butyl group, isobutyl group and so on. However, $R^3$ may combine with $R^4$ and $R^5$ may combine with $R^6$ to form a 5-member ring or a 6-member ring. In the latter case, the olefin of the formula (IX) is represented by the following formula (XII), (XIII), (XIV) or (XV). When each of $R^3$ and $R^4CH_2$ in the formula (IX) is a hydrogen atom, the aziridination proceeds with high enantioselectivity.

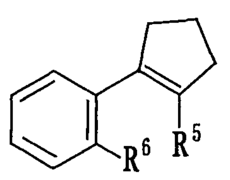

· · · · · (XII)

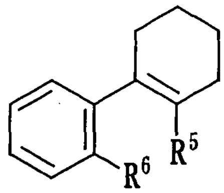

· · · · · (XIII)

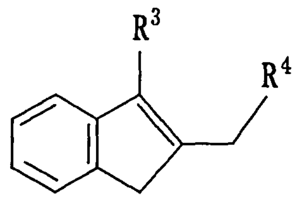

· · · · · (XIV)

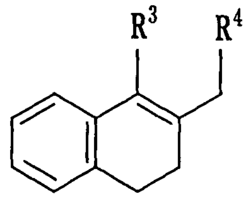

· · · · · (XV)

Concretely, the olefin of the formula (IX) includes an olefin represented by the above formula (X) and an olefin represented by the above formula (XI).

In the method of producing an optically active aziridine compound according to the invention, the olefin represented by the formula (IV) is used to produce an aziridine compound represented by the formula (I). On the other hand, in the method of producing an optically active amine compound according to the invention, the olefin represented by the formula (IX) is used to produce an amine compound represented by the formula (VIII). Moreover, both enantiomers of the optically active aziridine compound as a final product represented by the formula (I) can be obtained by properly using the Ru(salen)(CO) complex of the formula (II) and the Ru(salen)(CO) complex of the formula (III). Similarly, both enantiomers of the optically active amine compound as a final product represented by the formula (VIII) can be obtained by properly using the Ru(salen)(CO) complex of the formula (II) and the Ru(salen)(CO) complex of the formula (III). These optically active aziridine compounds and amine compounds can be used for the synthesis of medicines and agrochemicals.

In the invention, it is preferable to conduct the asymmetric aziridination or amination reaction in the presence of a zeolite. Although the asymmetric aziridination or amination reaction proceeds with good enatio-selectivity without the zeolite, the reaction yield can be largely improved by conducting the reactions in the presence of the zeolite. As the zeolite used in the invention, mention may be made of MS-3A, MS-4A, MS-5A and so on. Among them, MS-4A is preferable. An amount of the zeolite used is a range of 50 to 500 mg, preferably 100 to 300 mg per 1 mmol of the olefin as a substrate.

The asymmetric aziridination or amination reaction is carried out in a solvent. As the solvent, mention may be made of chlorobenzene, dichloroethane, toluene, dichloromethane and so on. Among them, dichloromethane is preferable. An amount of the solvent used is a range of 2 to 50 ml, preferably 4 to 10 ml per 1 mmol of the olefin as a substrate.

The aziridination and the amination according to the invention may be carried out at 10 to 40°C, preferably at room temperature. Since such a method can be carried out at room temperature, an energy cost for controlling the temperature can be suppressed. In the invention, the optically active aziridine compound or amine compound can be also produced by agitating a mixed solution of the olefin, the arylsulfonyl azide compound, the solvent and the catalyst. The reaction time is not particularly limited and is properly selected in accordance with the reaction temperature. It is preferable that when the reaction temperature is high, the reaction time is short, while when the reaction temperature is low, the reaction time is long. However, the reaction proceeds very slowly as the reaction temperature is lowered to

0°C.

The following examples are given in illustration of the invention and are not intended as limitations thereof.

(Synthesis Example 1 of Complex)

(1R, 2R)-1,2-diaminocyclohexane [Aldrich Chem. Co.] (27.5 mg, 0.24 mmol) is dissolved into ethanol (5 ml). The resulting solution is added to (aR)-3-formyl-2-hydroxy-2'-phenyl-1,1'-binaphthyl [which is synthesized according to the method described in H. Sasaki, R. Irie, T. Hamada, K. Suzuki and T. Katsuki, Tetrahedron, 50(41), 11827-11838 (1994) or the like] (179.7 mg, 0.48 mmol), and stirred at room temperature for 24 hours. After the completion of the reaction, the resulting precipitates are filtered and dried at 50°C under a reduced pressure for 1 hour. To the dried precipitates are added triruthenium dodecacarbonyl [Aldrich Chem. Co.] (152.1 mg, 0.24 mmol) and a dehydrated ethanol (10 ml) under a nitrogen atmosphere, and the resulting suspension is refluxed under heating for 5 days. After the temperature is turned to room temperature, the resulting reaction mixture is concentrated on a rotary evaporator to remove the solvent. The resulting residue is purified with a silica gel column using dichloromethane/ethanol (= 20/1) as a developing solvent to obtain a Ru(salen)(CO) complex (80.5 mg, yield: 36%) represented by the formula (VI).

The elementary analysis of the thus obtained complex shows H: 4.32%, C: 65.12% and N: 2.35%, which are well coincident with theoretical values of $C_{61}H_{44}N_2O_3Ru \cdot 2H_2O \cdot 2CH_2Cl_2$ (H: 4.52%, C: 65.23% and N: 2.42%). As a result of IR (KBr) measurement of the thus obtained complex, signals inherent to the complex are observed at 1323.1, 1423.4, 1490.9, 1541.0, 1577.7, 1612.4, 1934.5, 2019.3 $cm^{-1}$.

(Example 1)

A toluene solution of the complex of the formula (VI) (1.9 mg, 2.0 μmol) is concentrated twice azeotropically under vacuum. To the concentrated residue are added MS-4A (20 mg) and styrene (10.4 mg, 0.1 mmol), and further dichloromethane (0.5 ml) is added. The resulting suspension is agitated at room temperature for 0.5 hour, and then p-toluenesulfonyl azide (15.5 μl, 0.1 mmol) is added and further agitated for 24 hours. After the completion of the reaction, the reaction mixture is chromatographed on silica gel using hexane/ethyl acetate (= 1/0-19/1-8/2) to obtain the corresponding aziridine compound (19.4 mg, yield: 71%). As an enantiomeric excess of the aziridine compound is analyzed by a high performance liquid chromatography using a DAICEL CHIRALCEL OJ-H column and an eluant of hexane/isopropanol (= 1/1), it is 87%ee. The results are shown in Table 1.

(Examples 2 - 6)

The aziridination is carried out in the same manner as in Example 1 except that each of olefins shown in Table 1 is used instead of styrene. Moreover, the enantiomeric excess is analyzed by the high performance liquid chromatography using a DAICEL CHIRALPAK AS-H column and an eluant of hexane/isopropanol (= 2/1) in Example 2, a DAICEL CHIRALPAK AD-H column and an eluant of hexane/isopropanol (= 2/1) in Example 3, a DAICEL CHIRALPAK AS-H column and an eluant of hexane/isopropanol (= 7/3) in Example 4, a DAICEL CHIRALCEL OF column and an eluant of hexane/isopropanol (= 2/1) in Example 5, and a DAICEL CHIRALCEL OD-H column and an eluant of hexane/isopropanol (= 1/1) in Example 6. The reaction scheme is shown below, and the results are shown in Table 1.

Scheme

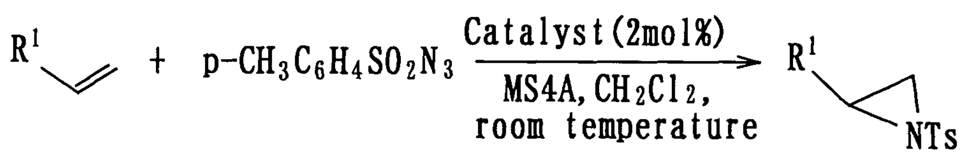

Table 1

| | Substrate | Yield (%) | Enantiometric excess (%ee) | Configuration |
|---|---|---|---|---|
| Example 1 | $C_6H_5-CH=CH_2$ | 71 | 87 | S * 1 |
| Example 2 | $p-BrC_6H_4-CH=CH_2$ | 87 | 90 | - |
| Example 3 | $p-O_2NC_6H_4-CH=CH_2$ | 98 | 92 | - |
| Example 4 | $C_6H_5CH\equiv C-CH=CH_2$ | 85 | 95 | - |
| Example 5 | $2-C_{10}H_7-CH=CH_2$ | 86 | 87 | - |
| Example 6 | $H_2C=C(C_6H_5)$ $-CH=CH_2$ | 7.3 | 90 | - |

* 1 Determined by comparing with the reported value of specific optical rotation. $[\alpha]_D^{22}$ of the Example 1 is +84.0 (c0.63, $CHCl_3$), while $[\alpha]_D$ of (S)-isomer (28%ee) described in M. Shi and C.-J. Wang, Chirality, 14, 412(2002) is +27.4 (c1, $CHCl_3$).

As seen from the results of Examples 1-6, the aziridine compound can be produced with high enantioselectivity by conducting the aziridination of the conjugated terminal olefin with the arylsulfonyl azide compound in the presence of the Ru(salen)(CO) complex.

(Examples 7 and 8)

The same procedure as in Example 1 is repeated except that each of olefins shown in Table 2 is used instead of styrene. Moreover, the enantiomeric excess is analyzed by the high performance liquid chromatography using a DAICEL CHIRALCEL OD-H column and an eluant of hexane/isopropanol (= 2/1) in Example 7 and a DAICEL CHIRALCEL OD-H column and an eluant of hexane/isopropanol (= 1/1) in Example 8. The results are shown in Table 2.

Table 2

| | Substrate | Product | Yield (%) | Enantiomeric excess (%ee) |
|---|---|---|---|---|
| Example 7 | 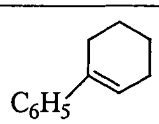 | 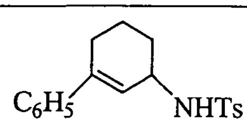 | 36 | 57 |
| Example 8 | 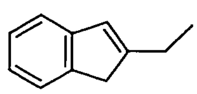 | 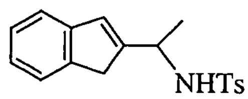 | 17 | 80 |

As seen from the results of Examples 7 and 8, the optically active amine compound is obtained by conducting the amination of the conjugated olefin having an allylic hydrogen with the arylsulfonyl azide compound in the presence of the Ru(salen)(CO) complex.

(Example 9)

The same procedure as in Example 1 is repeated except that the olefin shown in Table 3 is used instead of styrene. The result is shown in Table 3.

Table 3

| | Substrate | Product | Yield (%) | Enantiomeric excess (%ee) |
|---|---|---|---|---|
| Example 9 | 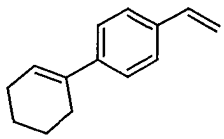 | 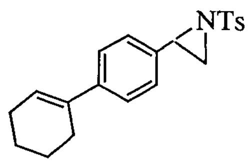 | 49 | 90 |
| | | 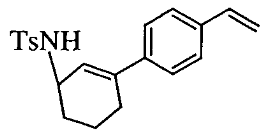 | 0 | - |

As seen from Example 9, the nitrene transfer reaction preferentially occurs in a mono-substituted double bond as compared with a tri-substituted double bond.

As mentioned above, according to the production method of the invention, the economical arylsulfonyl azide compound can be used as a nitrene precursor to attain the economization of the production process. Also, the optically active aziridine compound and amine compound can be produced from the olefins with high enantioselectivity , and both of enantiomers of these compounds can be obtained by properly selecting the catalyst. Thus, the optically active aziridine compound and amine compound to be used for the synthesis of medicines and agrochemicals can be provided at a state of a high optical purity.

## Claims

**1.** A method of producing an optically active aziridine compound represented by the following formula (I):

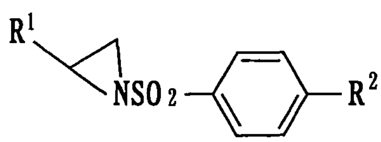 · · · · · (I)

(wherein $R^1$ is an alkenyl group having a carbon number of 2 to 20, an alkynyl group having a carbon number of 2 to 20 or an aryl group having a carbon number of 6 to 20 provided that a hydrogen atom in these alkenyl group, alkynyl group and aryl group may be substituted with a halogen atom or a nitro group, and $R^2$ is a hydrogen atom, a halogen atom, a substituted or non-substituted alkyl group having a carbon number of 1 to 4, or a substituted or non-substituted alkoxy group having a carbon number of 1 to 4), which comprises using as a catalyst an optically active Ru(salen)(CO) complex represented by the following formula (II) or (III):

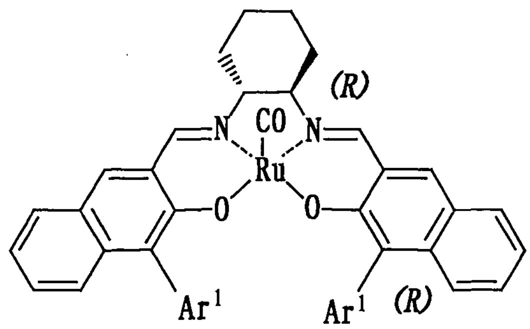

· · · · · (II)

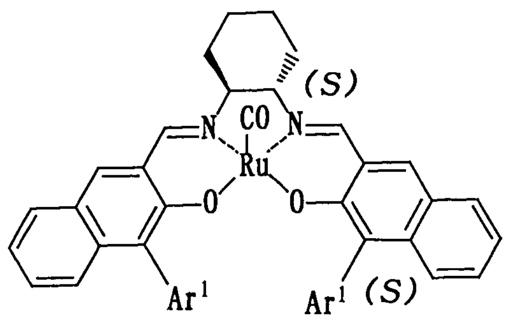

· · · · (III)

(wherein $Ar^1$ is independently an aryl group having a carbon number of 10 to 16), and subjecting an olefin represented by the following formula (IV):

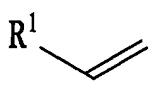

· · · · · (IV)

(wherein $R^1$ is the same meaning as mentioned above) to an asymmetric aziridination with an arylsulfonyl azide compound represented by the following formula (V):

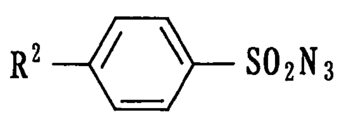

· · · · · (V)

(wherein $R^2$ is the same meaning as mentioned above).

**2.** A method according to claim 1, wherein the Ru(salen)(CO) complex is represented by the following formula (VI) or (VII).

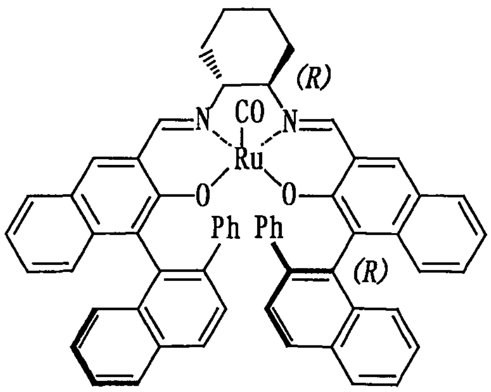

····· (VI)

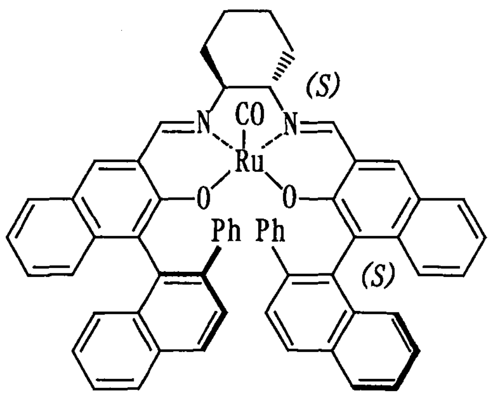

····· (VII)

**3.** A method according to claim 1, wherein $R^1$ in the olefin of the formula (IV) is phenyl group, p-bromophenyl group, p-nitrophenyl group, phenylethynyl group, 2-naphthyl group, 1-phenylvinyl group or p-(1-cyclohexenyl)-phenyl group.

**4.** A method according to claim 1, wherein the arylsulfonyl azide compound of the formula (V) is p-toluenesulfonyl azide (p-$CH_3C_6H_4SO_2N_3$).

**5.** A method of producing an optically active amine compound represented by the following formula (VIII):

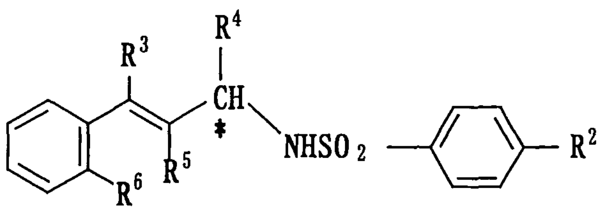

····· (VIII)

(wherein $R^2$ is a hydrogen atom, a halogen atom, a substituted or non-substituted alkyl group having a carbon number of 1 to 4, or a substituted or non-substituted alkoxy group having a carbon number of 1 to 4, and $R^3$, $R^4$, $R^5$ and $R^6$ are independently a hydrogen atom, or a linear or branched alkyl group having a carbon number of 1 to 20, provided that $R^3$ may combine with $R^4$ and $R^5$ may combine with $R^6$ to form a 5-member ring or a 6-member ring), which comprises using as a catalyst an optically active Ru(salen)(CO) complex represented by the following formula (II) or (III):

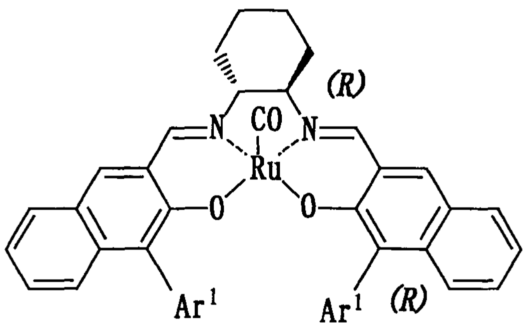

······ (II)

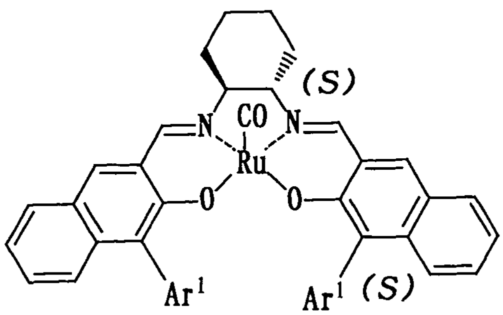

······ (III)

(wherein $Ar^1$ is independently an aryl group having a carbon number of 10 to 16), and subjecting an olefin expressed by the following formula (IX):

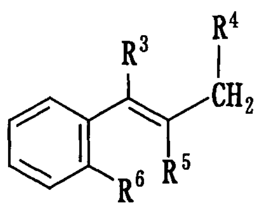

······ (IX)

(wherein each of $R^3$, $R^4$, $R^5$ and $R^6$ is the same meaning as mentioned above) to an asymmetric amination with an arylsulfonyl azide compound expressed by the following formula (V):

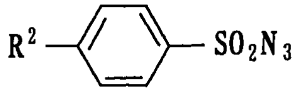

······ (V)

(wherein $R^2$ is the same meaning as mentioned above).

**6.** A method according to claim 5, wherein the Ru(salen)(CO) complex is represented by the following formula (VI) or (VII).

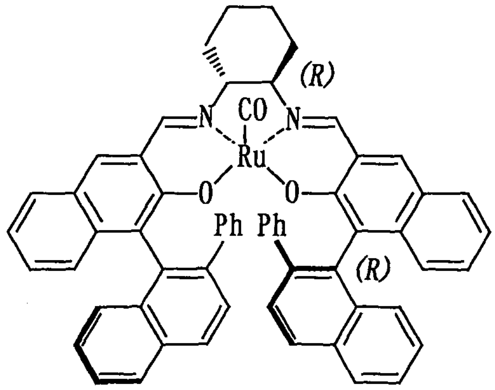

· · · · · (VI)

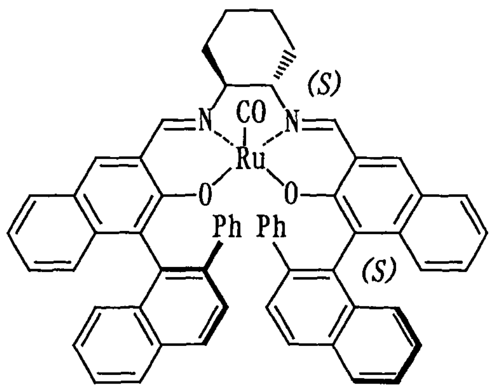

· · · · · (VII)

7. A method according to claim 5, wherein the olefin of the formula (IX) is represented by the following formula (X) or (XI).

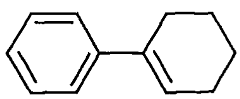

· · · · · (X)

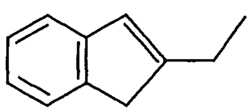

· · · · · (XI)

8. A method according to claim 5, wherein the arylsulfonyl azide compound of the formula (V) is p-toluenesulfonyl azide ($p\text{-}CH_3C_6H_4SO_2N_3$).

European Patent Office

# EUROPEAN SEARCH REPORT

Application Number
EP 04 25 0770

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 02/076932 A (JAPAN SCIENCE & TECH CORP ; KOHMURA YOSHINORI (JP); KATSUKI TSUTOMU (J) 3 October 2002 (2002-10-03)<br>& EP 1 375 478 A<br>2 January 2004 (2004-01-02)<br>* paragraph [0014] - paragraph [0022]; example 1 *<br>----- | 1-8 | C07D203/24 |
| A | MURAKAMI M ET AL: "Ru(salen)-catalyzed asymmetric sulfimidation using arylsulfonyl azide"<br>TETRAHEDRON LETTERS, ELSEVIER SCIENCE PUBLISHERS, AMSTERDAM, NL,<br>vol. 42, no. 40,<br>1 October 2001 (2001-10-01), pages 7071-7074, XP004317894<br>ISSN: 0040-4039<br>Scheme 2<br>----- | 1-8 | |
| A | WARREN B K ET AL: "Some Reactions of 1,4-Dihydropyridines with Organic Azides. Synthesis of 2,7-Diazabicyclo[4.1.0]hept-3-enes with Analgesic and Antiprotozola actiivty"<br>J.MED.CHEM.,<br>vol. 24, no. 4, 1981, pages 462-464, XP002277377<br>Scheme I<br>----- | 1-8 | TECHNICAL FIELDS SEARCHED (Int.Cl.7)<br>C07D |
| P,X | KAZUFUMI O ET AL: "Enantioselective Aziridination and Amination Using p-Toluenesulfonyl azide in the Presence of Ru(salen)(CO) Complex"<br>CHEMISTRY LETTERS,<br>vol. 32, no. 4, April 2003 (2003-04), pages 354-355, XP0009029644<br>Schemes 1,2* tables 1,2 *<br>----- | 1-8 | |

The present search report has been drawn up for all claims

| Place of search | Date of completion of the search | Examiner |
|---|---|---|
| Munich | 20 April 2004 | Usuelli, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 25 0770

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2004

| Patent document cited in search report | | Publication date | Patent family member(s) | | | Publication date |
|---|---|---|---|---|---|---|
| WO 02076932 | A | 03-10-2002 | JP | 2002284758 | A | 03-10-2002 |
| | | | EP | 1375478 | A1 | 02-01-2004 |
| | | | WO | 02076932 | A1 | 03-10-2002 |
| | | | US | 2003139627 | A1 | 24-07-2003 |
| EP 1375478 | A | 02-01-2004 | JP | 2002284758 | A | 03-10-2002 |
| | | | EP | 1375478 | A1 | 02-01-2004 |
| | | | WO | 02076932 | A1 | 03-10-2002 |
| | | | US | 2003139627 | A1 | 24-07-2003 |

EPO FORM P0459

For more details about this annex : see Official Journal of the European Patent Office, No. 12/82